# FASCICULE DE BREVET EUROPEEN

(11) **EP 1 491 819 B1**
(45) Date de publication et mention de la délivrance du brevet: **24.08.2005**
(21) Numéro de dépôt: 04291189.1
(22) Date de dépôt: 07.05.2004
(51) Int. Cl.: F23D 3/24, A01M 13/00, A01M 1/20

(54) **Système de brûleur à combustion catalytique et flacon équipé d'un tel système**
Brennersystem mit katalytischer Verbrennung und Fläschchen mit einem solchen System
Burner System with Catalytic Combustion and Small Bottle with such a System

(30) Priorité: 27.06.2003 FR 0307767
(43) Date de publication de la demande: 29.12.2004
(73) Titulaire: PRODUITS BERGER, 75116 Paris (FR)
(72) Inventeur: Gomez, Corinne, 27400 Incarville (FR); Lehoux, Jannick, 27310 Thuit Signol (FR)
(74) Mandataire: Bentz, Jean-Paul

(56) Documents cités:
- EP-A- 0 277 875
- EP-A- 1 214 948
- WO-A-99/63267
- FR-A- 2 483 782
- FR-A- 2 680 118

## Description

La présente invention concerne un système de brûleur à combustion catalytique comprenant un brûleur à combustion catalytique et constituant un perfectionnement des brûleurs à combustion catalytique connus à ce jour. Elle concerne également un flacon équipé d'un tel système de brûleur.

Les brûleurs à combustion catalytique utilisés à ce jour sont réalisés en matière poreuse et comportent, au niveau de leur partie supérieure, une zone périphérique, par exemple annulaire, qui supporte un catalyseur et une zone centrale sans catalyseur formant zone de vaporisation.

A titre d'exemples, on peut citer les brûleurs décrits dans les documents EP 0 277 875 B1 et WO 99/63267 au nom de la demanderesse.

Ce type de brûleur muni d'une mèche est de manière courante disposé sur le goulot d'un flacon contenant la composition combustible, la mèche trempant dans ladite composition combustible.

La composition combustible amenée par là mèche pénètre par capillarité dans les pores de la matière poreuse du brûleur.

Une partie de cette composition parvient ainsi dans la zone périphérique supportant le catalyseur et y subit une combustion catalytique qui maintient cette zone périphérique à une température élevée.

Une autre partie de cette composition combustible traverse la zone centrale sans catalyseur et y subit une diffusion par vaporisation.

On constate toutefois, que cette diffusion jusqu'aux zones périphérique et centrale n'est pas assurée de manière constante au cours des utilisations et qu'elle dépend, dans les faits, étroitement du niveau de remplissage du flacon en composition combustible.

Cette diffusion peut être également altérée, le cas échéant, à la suite d'une éventuelle carbonisation de la mèche qui a pu produire des particules pouvant boucher certains des pores du brûleur.

Il est par expérience admis qu'une diffusion, pour observer une désodorisation et une purification de l'air ambiant, est satisfaisante pour une consommation en composition combustible qui est régulière et constante.

Or, si des valeurs de consommation régulières et constantes sont effectivement atteintes avec les brûleurs à combustion catalytique actuels, elles le sont principalement pour des niveaux de remplissage du flacon de l'ordre des deux tiers en volume de composition combustible contenu dans le flacon.

Par contre, en deçà et au-delà de ce niveau de remplissage des deux tiers, on constate une variation importante de la consommation en composition combustible.

Lorsque le flacon est entièrement rempli de composition combustible, l'apport en composition combustible au niveau du brûleur est trop important et peut avoir pour effet de limiter le fonctionnement du brûleur, voire de provoquer son arrêt.

Au contraire, lorsque le flacon contient au plus un quart en volume de composition combustible, la mèche, qui est couramment réalisée en coton, ne permet plus d'assurer la diffusion, dans des conditions de fonctionnement satisfaisantes, de la composition combustible au niveau du brûleur. De faibles consommations sont alors observées.

En outre, lorsque le brûleur est maintenu en fonctionnement jusqu'à épuisement de la composition combustible, la mèche s'altère par carbonisation. Des utilisations répétées dans de telles conditions peuvent encore accélérer le vieillissement du brûleur.

On constate donc, dans tous les cas, que le flacon ne soit pas assez rempli ou au contraire qu'il le soit complètement, que la consommation en composition combustible varie de manière significative et limite, de ce fait, les performances en qualité de parfumage, de désinfection et de destruction des molécules et qu'une détérioration du brûleur peut intervenir.

Le but de la présente invention est donc de remédier à l'ensemble des inconvénients précités lors de la mise en oeuvre des brûleurs connus et de proposer un système de brûleur à combustion catalytique permettant une utilisation satisfaisante et optimale d'un flacon doté d'un tel système de brûleur, indépendamment du niveau de remplissage en composition combustible du dit flacon et, par voie de conséquence, d'assurer un fonctionnement sensiblement régulier et optimal du brûleur à combustion catalytique, sans risque de carbonisation de la mèche, quel que soit le niveau de composition combustible à l'intérieur du flacon.

Selon l'invention, ce système comprend en outre un manchon en une seconde matière poreuse, ledit manchon comportant une cavité sensiblement axiale adaptée à enserrer une mèche destinée à amener au brûleur une composition combustible, ledit manchon étant disposé dans le prolongement de la partie inférieure du brûleur, de manière telle que la composition combustible peut migrer des pores de la partie supérieure du manchon vers les pores de la partie inférieure du brûleur.

La présence de ce manchon, qui vient en complément des brûleurs actuels, permet de contrôler l'apport en composition combustible au niveau du brûleur.

Ainsi, le fait que la partie supérieure de la mèche soit enserrée par le manchon permet de créer une limitation de l'apport en composition combustible au niveau du brûleur par la mèche, lorsque le flacon est rempli de composition combustible, sur toute sa hauteur ou sur une partie importante de cette hauteur.

Par ailleurs, la capillarité du manchon s'ajoute à la capillarité de la mèche pour aspirer une quantité de liquide combustible plus importante que celle qui serait aspirée par la mèche seule, lorsque la quantité de liquide combustible contenue dans le flacon est faible.

En outre, la chaleur transmise par le brûleur au manchon participe à la migration ou à l'entraînement de la composition combustible à travers la mèche vers le brûleur à combustion catalytique.

Ainsi, grâce à la matière poreuse formant le manchon, l'apport de composition combustible au niveau du brûleur est assuré et régulé, même dans le cas de faibles niveaux de remplissage du flacon.

On utilise, pour le manchon, une seconde matière poreuse présentant une porosité de préférence inférieure à la porosité de la première matière poreuse formant le brûleur.

Il est bien évidemment possible d'adapter et de régler l'apport de composition combustible au niveau du brûleur en faisant varier la porosité du brûleur d'une part et celle du manchon d'autre part.

Dans tous les cas, la capillarité radiale et longitudinale de la mèche est très supérieure à celle du manchon.

Ainsi, et jusqu'à épuisement de la composition combustible, le brûleur est approvisionné en composition combustible, par capillarité à partir de la mèche ainsi qu'à partir du manchon.

Un autre avantage réside dans le choix élargi du type de mèche utilisable dans un tel système de brûleur.

Avec les brûleurs actuels, le choix de la mèche est déterminé en fonction de plusieurs paramètres que sont notamment la matière de la mèche, le nombre de fils qui la constituent ainsi que son mode de tressage.

Le contrôle de l'apport en composition combustible au niveau du brûleur étant assuré au moyen du manchon, l'incidence de la mèche, que ce soit en termes de structure, de matière ou de forme, est moindre.

L'invention concerne également un flacon à combustion catalytique, adapté à contenir une composition combustible et à recevoir au niveau de son goulot un système de brûleur à combustion catalytique selon l'invention, ce système étant adapté à recevoir une mèche trempant dans ladite composition combustible.

Grâce au système de brûleur à combustion catalytique selon l'invention, et en particulier à la présence du manchon disposé dans le prolongement du brûleur à combustion catalytique de structure connue, on assure un positionnement nécessairement correct du système de brûleur dans le goulot du flacon. En effet, le manchon joue le rôle de détrompeur et impose le positionnement correct et adéquat du système de brûleur dans le goulot du flacon.

D'autres avantages et particularités de l'invention résulteront de la description qui va suivre, donnée à titre d'exemples non limitatifs et faite en référence aux figures annexées dans lesquelles :
- la figure 1 est une vue schématique en élévation d'un flacon équipé d'un système de brûleur à combustion catalytique conforme à la présente invention ;
- la figure 2 est une vue schématique agrandie en coupe axiale du système de brûleur représenté à la figure 1, dans un premier mode de réalisation de la présente invention, pourvu d'une mèche et adapté à équiper le flacon de la figure 1 ;
- la figure 3 est une vue semblable à la figure 2 d'un second mode de réalisation d'un système de brûleur selon la présente invention ;
- la figure 4 est une vue semblable à la figure 2 d'un troisième mode de réalisation d'un système de brûleur selon la présente invention ; et
- la figure 5 est une vue semblable à la figure 1 d'un flacon équipé d'un système de brûleur à combustion catalytique selon un quatrième mode de réalisation, ledit système étant disposé sur une embase.

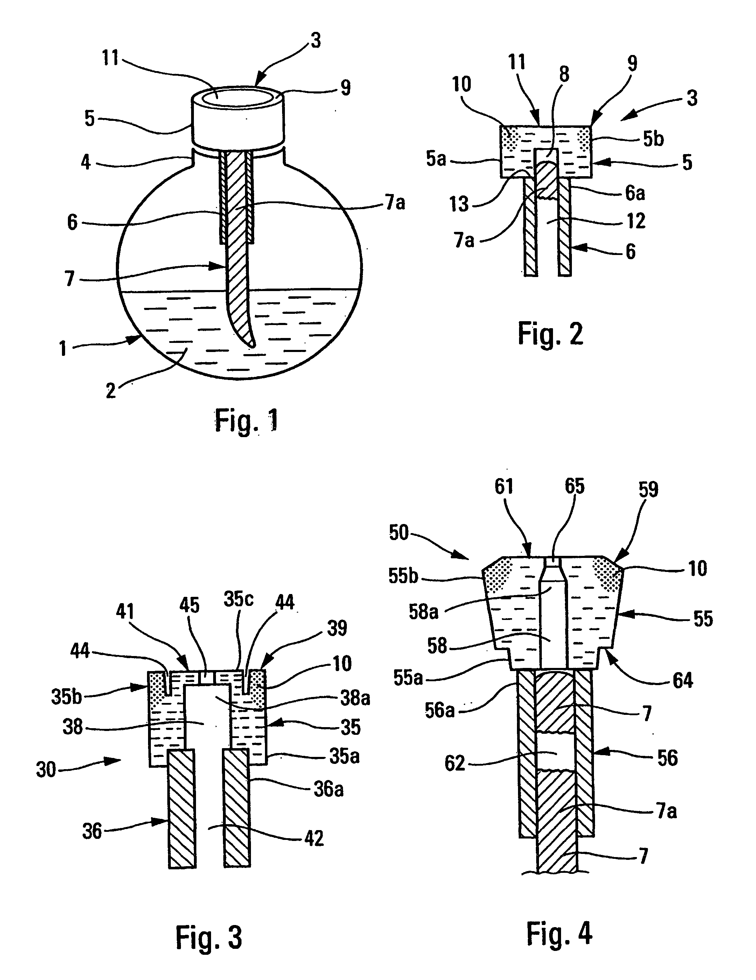

Les éléments communs aux figures 1 à 5 sont identifiés par des références numériques identiques.

Sur la figure 1, on a représenté un flacon à combustion catalytique 1 adapté à contenir une composition combustible 2 et à recevoir, à sa partie supérieure, un système de brûleur à combustion catalytique 3 conforme à l'invention.

Ce flacon 1 peut être de forme quelconque et présente un goulot 4 apte à recevoir le système de brûleur à combustion catalytique 3.

Le système de brûleur à combustion catalytique 3 peut être doté, à sa partie inférieure, d'un support (non représenté) permettant son introduction, soit directement dans le goulot 4 du flacon 1, soit dans le trou central d'une embase (non représentée), cette dernière étant adaptée à être fixée au niveau du goulot 4 du flacon 1.

Des support et embase convenant pour la fixation, sur le flacon 1, d'un tel système de brûleur 3 ont notamment été décrits dans la demande WO 99/63267.

La composition combustible 2 est un liquide combustible approprié, conforme aux réglementations en vigueur et adapté à la combustion catalytique et à la vaporisation.

Cette composition combustible 2 peut notamment être un alcool, en particulier l'alcool isopropylique, et peut comprendre en outre une matière parfumée et/ou une matière active.

Ce système de brûleur 3 est formé d'un brûleur à combustion catalytique 5 et d'un manchon 6, et est adapté à recevoir une mèche 7 trempant dans la composition combustible 2.

Comme représenté en détail à la figure 2, le brûleur 5 comporte, à sa partie inférieure 5a, une cavité 8 sensiblement axiale pourvue de la mèche 7 destinée à amener au brûleur 5 la composition combustible 2.

Le brûleur 5 comporte à sa partie supérieure 5b une zone périphérique 9, de forme annulaire, qui supporte un catalyseur 10 et une zone centrale 11 sans catalyseur formant zone de vaporisation.

Ce brûleur 5 est réalisé en une première matière poreuse appropriée et adaptée à résister aux températures d'au moins 400°C atteintes dans la zone supportant le catalyseur pendant le fonctionnement du brûleur. Cette matière peut notamment être une matière céramique, et par exemple préparée à partir de kaolin ou de cordiérite.

Le catalyseur est par exemple un métal appartenant au groupe VIII du tableau de classification périodique des éléments.

Le manchon 6 du système de brûleur à combustion catalytique 3 selon l'invention comporte une cavité 12 sensiblement axiale adaptée à enserrer la mèche 7 (non représentée sur la figure 2) de manière telle que la composition combustible 2 peut migrer des pores de la partie supérieure 6a du manchon 6 vers les pores de la partie inférieure 5a du brûleur 5.

Le manchon 6 est disposé dans le prolongement de la partie inférieure 5a du brûleur 5, de manière à aligner sensiblement co-axialement, sur une partie au moins de sa surface, la cavité 8 du brûleur 5 avec la cavité 12 du manchon 6.

Pour assurer la migration de la composition combustible 2 des pores de la partie supérieure 6a du manchon 6 vers les pores de la partie inférieure 5a du brûleur 5, le manchon 6 est réalisé en une seconde matière poreuse, qui peut notamment être une matière céramique, par exemple préparée à partir de kaolin ou de cordiérite.

De préférence, la porosité de la seconde matière poreuse est inférieure à la porosité de la première matière poreuse formant le brûleur 5.

Habituellement, les première et seconde matières poreuses respectivement du brûleur 5 et du manchon 6, sont préparées à partir de cordiérite.

La partie inférieure 5a du brûleur 5 et la partie supérieure 6a du manchon 6 présentent des formes respectives complémentaires l'une de l'autre.

Sur la figure 2, les parties 5a et 6a présentent une surface de jonction 13 plane.

Il est cependant possible de prévoir, au lieu d'une surface de jonction plane, un mode de jonction par emboîtement, dans la mesure où la forme retenue pour les parties inférieure 5a du brûleur 5 et supérieure 6a du manchon 6 permet d'assurer la migration de la composition combustible 2 du manchon 6 vers le brûleur 5.

Le brûleur 5 et le manchon 6 sont par exemple assemblés au moyen d'un scellement poreux adapté à assurer la migration de la composition combustible du manchon 6 vers le brûleur 5.

Dans une version avantageuse de réalisation du système de brûleur 3, qui évite notamment l'opération de scellement mentionnée précédemment, le brûleur 5 et le manchon 6 constituent une seule et unique pièce.

Le procédé de fabrication du système de brûleur est en conséquence adapté pour permettre la réalisation, le cas échéant, de porosités distinctes entre la partie du système 3 correspondant au brûleur 5 d'une part et la partie du système 3 correspondant au manchon 6 d'autre part.

Une autre variante du système de brûleur à combustion catalytique selon l'invention est représentée à la figure 3.

Ce système de brûleur 30 comprend un brûleur à combustion catalytique 35 et un manchon 36 joints par emboîtement, de préférence complété par un scellement poreux, au niveau de la partie inférieure 35a du brûleur 35 et de la partie supérieure 36a du manchon 36.

La cavité 42 du manchon 36 est située dans le prolongement de la cavité 38 du brûleur 35 et présente dans le cas représenté à la figure 3 une section transversale plus faible que la section transversale correspondante de la cavité 42 du manchon 36.

Le brûleur 35 présente une gorge annulaire 44 sensiblement axiale s'étendant depuis la surface supérieure 35c du brûleur 35 et séparant la zone périphérique 39 supportant le catalyseur 10 de la zone centrale 41 sans catalyseur formant zone de vaporisation.

Cette structure particulière de la partie supérieure 35b d'un tel brûleur 35 a notamment été décrite dans le brevet EP 0 277 875 B1 au nom de la demanderesse.

Le brûleur 35 présente en outre un canal 45 pour mettre à l'atmosphère la partie supérieure 38a de la cavité 38 destinée à recevoir la mèche.

Dans l'exemple représenté à la figure 3, le canal 45 est ménagé sensiblement axialement. Toutefois, rien n'interdit d'envisager d'autres dispositions pour ce canal 45, en particulier la disposition particulière représentée à la figure 8 du document WO 99/63267.

Une troisième variante de réalisation d'un système de brûleur conforme à l'invention est représentée à la figure 4.

Le système de brûleur 50 représenté à la figure 4 comporte un brûleur à combustion catalytique 55 et un manchon 56.

Ce manchon 56 est disposé dans le prolongement de la partie inférieure 55a du brûleur 55 et est en contact, au niveau de sa partie supérieure 56a, avec la partie inférieure 55a du brûleur 55.

Ce brûleur 55 présente une forme tronconique évasée vers le haut.

La partie supérieure 55b du brûleur 55 comporte une zone périphérique 59 supportant un catalyseur 10 et qui entoure une zone centrale 61 sans catalyseur, ainsi qu'une cavité 58.

Cette cavité 58 est reliée à l'atmosphère, au niveau de sa partie supérieure 58a, par le canal 65 situé au centre de la zone centrale 61.

La cavité 62 du manchon 56 peut présenter une section transversale plus étroite que la section transversale correspondante de la cavité 58 du brûleur 55.

La cavité 62 du manchon 58 est ici entièrement remplie par la mèche 7 qui ne pénètre pas dans la cavité 58 du brûleur 55.

Cette forme tronconique particulière du brûleur permet d'augmenter la surface de vaporisation de la composition combustible et ainsi d'assurer une meilleure diffusion de cette composition dans l'air ambiant d'une enceinte ou pièce.

Le brûleur 55 peut avantageusement être muni d'au moins un épaulement 64 destiné à délimiter la partie de la zone périphérique 59 qui est destinée à recevoir le catalyseur 10.

Accessoirement, un tel épaulement 64 coopère avec le manchon 56 pour permettre un positionnement ajusté du système de brûleur 50 selon l'invention sur le flacon 1 de la figure 1, au niveau de son goulot 4.

Dans les exemples de réalisation qui viennent d'être décrits ci-dessus mais également de manière générale, la mèche 7 est une mèche connue quelconque, par exemple une mèche en coton.

On peut tout à fait envisager d'utiliser une mèche en matière minérale, telle qu'une mèche en fibres minérales.

La mèche 7 est choisie de telle sorte que sa capillarité est très supérieure à la capillarité radiale et longitudinale du manchon 6.

La mèche 7 est disposée dans le flacon à combustion catalytique de façon à tremper dans la composition combustible 2 et est maintenue, dans le système de brûleur 3, 30, 50, par sa partie supérieure 7a qui est enserrée dans le manchon 6, 36, 56.

Le manchon 6, 36, 56 recouvre au moins 10 %, généralement entre 10 et 40%, avantageusement entre 20 et 30 %, et de préférence de l'ordre de 25 % de la longueur de la mèche 7.

La dimension du manchon 6, 36, 56 dépend à la fois de la porosité de la seconde matière poreuse formant le manchon 6, 36, 56 et du degré de serrage de la mèche 7 dans ledit manchon 6, 36, 56, en particulier lorsque la mèche utilisée est en coton.

De manière classique, la mèche 7 occupe l'ensemble du volume de la cavité 12, 42, 62 du manchon 6, 36, 56.

Mais on peut également prévoir que ladite mèche 7 occupe en outre tout ou partie du volume de la cavité 8, 38, 58 du brûleur 5, 35, 55.

Dans cette dernière hypothèse, en particulier lorsque tout le volume de la cavité 8, 38, 58 du brûleur 5, 35, 55 est également occupé par l'extrémité supérieure de la mèche 7, il est préférable de pourvoir le brûleur 5, 35, 55, d'un canal 45, 65 de mise à l'atmosphère de ladite cavité 8, 38, 58, pour éviter tout risque de carbonisation de la mèche 7.

En tout état de cause, il est à noter que, dans le cas de l'utilisation d'un système de brûleur 3, 30, 50, dans lequel la partie supérieure 7a de la mèche 7 occupe la cavité 12, 42, 62 du manchon 6, 36, 56 mais non la cavité 8, 38, 58 du brûleur 5, 35, 55, il ne se produit pas de carbonisation de la mèche 7, indépendamment de la présence ou non d'un canal 45, 65 au niveau du brûleur 5, 35, 55.

Il est également possible de prévoir un système de brûleur comportant un brûleur de structure très simple.

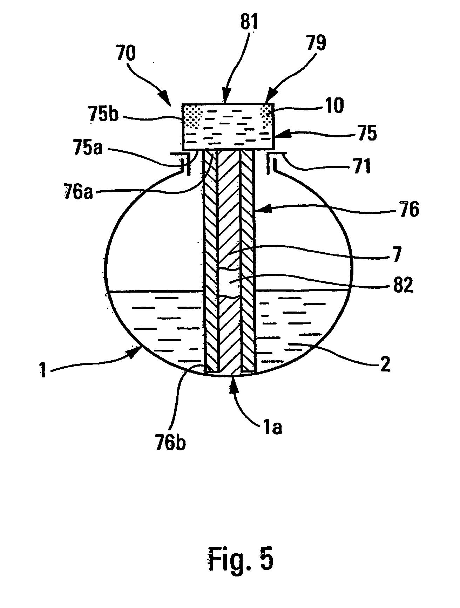

C'est la cas de la quatrième variante de réalisation d'un système de brûleur conforme à l'invention tel que représenté à la figure 5.

Le système de brûleur 70 représenté à la figure 5 comprend un brûleur à combustion catalytique 75 et un manchon 76 joints par scellement au niveau de la partie inférieure 75a du brûleur 75 et de la partie supérieure 76a du manchon 76.

Le brûleur 75 comporte une zone périphérique (...) 79 supportant un catalyseur 10, cette zone 79 entourant une zone centrale 81 sans catalyseur.

Par contre, et contrairement à toutes les structures de brûleurs 5, 35 et 55 vues précédemment et représentées aux figures 2 à 4, le brûleur 75 a une structure pleine et ne présente aucune cavité au niveau de sa partie inférieure 75a.

Ce brûleur 75 est disposé sur un support et/ou embase 71 fixé au goulot 4 du flacon 1.

Dans cette réalisation particulière du système de brûleur 70 représenté à la figure 5, le manchon 76 occupe sensiblement toute la hauteur du flacon 1. La partie inférieure 76b du manchon 76 est à proximité du fond 1a du flacon 1.

Il est par exemple possible d'envisager que le manchon 76 ait une structure télescopique afin que sa longueur s'adapte aux différentes hauteurs des flacons sur lesquels le système de brûleur 70 est destiné à être utilisé.

En outre, sur cette même figure 5, la totalité de la cavité 82 du manchon 76 est occupée par la mèche 7.

On pourrait également utiliser le brûleur 75 avec le manchon 76, sans aucune mèche.

Bien entendu, la présente invention n'est pas limitée aux exemples de réalisation qui viennent d'être décrits, et on peut apporter à ceux-ci de nombreux changements et modifications sans sortir du domaine de la présente invention.

On peut notamment combiner de manière quelconque les éléments formant le système de brûleur à combustion catalytique 3, 30, 50, 70, à savoir le brûleur à combustion catalytique 5, 35, 55, 75 et le manchon 6, 36, 56, 76.

On peut en particulier prévoir de doter la partie supérieure 5b, 55b et 75b des brûleurs 5, 55 et 75 représentés respectivement aux figures 2, 4 et 5, d'une gorge annulaire séparant respectivement les zones périphériques 9, 59, 79 des zones centrales 11, 61, 81, une telle gorge présentant les caractéristiques de la gorge annulaire 44 représentée à la figure 3.

Il est également tout à fait possible de prévoir que la partie supérieure de la cavité 8 du brûleur 5 de la figure 2 soit reliée à l'atmosphère par un canal, tels que ceux représentés aux figures 3 et 4 et comportant respectivement les références 45 et 65.

Par ailleurs, la forme du brûleur peut être quelconque, et ne doit pas être interprétée comme étant seulement limitée aux formes cylindrique et tronconique représentées.

Rien n'interdit en outre d'envisager la réalisation d'un système de brûleur à combustion catalytique selon l'invention à partir d'un brûleur déjà connu et disponible à ce jour dans le commerce, auquel est rapporté un manchon 6, 36, 56, 76, sous réserve que ce dernier réponde aux caractéristiques énoncées ci-dessus.

## Revendications

1. Système de brûleur à combustion catalytique comprenant un brûleur (5, 35, 55, 75) en une première matière poreuse comportant à sa partie supérieure (5b, 35b, 55b, 75b) une zone périphérique (9, 39, 59, 79) supportant un catalyseur (10) et une zone centrale (11, 41, 61, 81) sans catalyseur formant zone de vaporisation, **caractérisé en ce que** ce système (3, 30, 50, 70) comprend en outre un manchon (6, 36, 56, 76) en une seconde matière poreuse comportant une cavité (12, 42, 62, 82) sensiblement axiale adaptée à enserrer une mèche (7, 7a) destinée à amener au brûleur (5, 35, 55, 75) une composition combustible (2), ledit manchon (6, 36, 56, 76) étant disposé dans le prolongement de la partie inférieure (5a, 35a, 55a, 75a) du brûleur (5, 35, 55, 75), de manière telle que la composition combustible (2) peut migrer des pores de la partie supérieure (6a, 36a, 56a, 76a) du manchon (6, 36, 56, 76) vers les pores de la partie inférieure (5a, 35a, 55a, 75a) du brûleur (5, 35, 55, 75).

2. Système selon la revendication 1, **caractérisé en ce que** la porosité de la seconde matière poreuse du manchon (6, 36, 56, 76) est de préférence inférieure à la porosité de la première matière poreuse du brûleur (5, 35, 55, 75).

3. Système selon la revendication 1 ou 2, **caractérisé en ce que** le manchon (6, 36, 56, 76) recouvre au moins 10 %, généralement entre 10 et 40%, avantageusement entre 20 et 30 %, et de préférence de l'ordre de 25 %, de la longueur de la mèche (7, 7a).

4. Système selon l'une quelconque des revendications 1 à 3, **caractérisé en ce que** le brûleur (5, 35, 55, 75) et le manchon (6, 36, 56, 76) sont assemblés au moyen d'un scellement poreux adapté à assurer la migration de la composition combustible (2) du manchon (6, 36, 56, 76) vers le brûleur (5, 35, 55, 75).

5. Système selon l'une quelconque des revendications 1 à 3, **caractérisé en ce que** le brûleur (5, 35, 55, 75) et le manchon (6, 36, 56, 76) constituent une seule pièce.

6. Système selon l'une quelconque des revendications 1 à 5, **caractérisé en ce que** les première et seconde matières poreuses sont constituées par de la cordiérite.

7. Système selon l'une quelconque des revendications 1 à 6, **caractérisé en ce que** le brûleur (35) présente une gorge annulaire (44) sensiblement axiale s'étendant depuis la surface supérieure (35c) du brûleur (35) et séparant la zone périphérique (39) supportant le catalyseur (10) de la zone centrale (41) sans catalyseur formant zone de vaporisation.

8. Système selon l'une quelconque des revendications 1 à 7, **caractérisé en ce que** le brûleur (5, 35, 55) comporte, à sa partie inférieure (5a, 35a, 55a), une cavité (8, 38, 58) sensiblement axiale adaptée à recevoir la mèche (7, 7a).

9. Système selon la revendication 8, **caractérisé en ce que** le brûleur (35, 55) présente un canal (45, 65) pour mettre à l'atmosphère la partie supérieure (38a, 58a) de la cavité (38, 58) recevant la mèche (7, 7a).

10. Système selon la revendication 7 ou 8, le système (3, 30, 50) étant équipé d'une mèche (7, 7a), **caractérisé en ce que** la mèche (7, 7a) est insérée dans la cavité (12, 42, 62) sensiblement axiale du manchon (6, 36, 56) et, le cas échéant, dans tout ou partie de la cavité (8, 38, 58) sensiblement axiale du brûleur (5, 35, 55).

11. Flacon à combustion catalytique, adapté à contenir une composition combustible (2) et à recevoir au niveau de son goulot (4) un système de brûleur à combustion catalytique (3, 30, 50, 70) adapté à recevoir une mèche (7, 7a) trempant dans ladite composition combustible (2), **caractérisé en ce qu'**il est équipé d'un système de brûleur (3, 30, 50, 70) selon l'une quelconque des revendications 1 à 10.

## Patentansprüche

1. Brennersystem zur katalytischen Verbrennung, umfassend einen Brenner (5, 35, 55, 75) aus einem ersten porösen Material, der an seinem oberen Teil (5b, 35b, 55b, 75b) eine Umfangszone (9, 39, 59, 79), die einen Katalysator trägt (10), und eine Zentralzone (11, 41, 61, 81) ohne Katalysator, die eine Verdampfungszone bildet, aufweist, **dadurch gekennzeichnet, dass** dieses System (3, 30, 50, 70) ferner eine Hülse (6, 36, 56, 76) aus einem zweiten porösen Material umfasst, die einen im Wesentlichen axialen Hohlraum (12, 42, 62, 82) aufweist, der so ausgebildet ist, dass dieser einen Docht (7, 7a) einspannt, der dazu bestimmt ist, dem Brenner (5, 35, 55, 75) eine brennbare Zusammensetzung (2) zuzuführen, wobei die Hülse (6, 36, 56, 76) in der Verlängerung des unteren Teils (5a, 35a, 55a, 75a) des Brenners (5, 35, 55, 75) angeordnet ist, derart, dass die brennbare Zusammensetzung (2) aus den Poren des oberen Teils (6a, 36a, 56a, 76a) der Hülse (6, 36, 56, 76) in Richtung der Poren des unteren Teils (5a, 35a, 55a, 75a) des Brenners (5, 35, 55, 75) migrieren kann.

2. System nach Anspruch 1, **dadurch gekennzeichnet, dass** die Porosität des zweiten porösen Materials der Hülse (6, 36, 56, 76) vorzugsweise geringer ist als die Porosität des ersten porösen Materials des Brenners (5, 35, 55, 75).

3. System nach Anspruch 1 oder 2, **dadurch gekennzeichnet, dass** die Hülse (6, 36, 56, 76) wenigstens 10%, im Wesentlichen zwischen 10 und 40%, in vorteilhafter Weise zwischen 20 und 30% und vorzugsweise in der Größenordnung von 25% der Länge der Hülse (7, 7a) abdeckt.

4. System nach einem der Ansprüche 1 bis 3, **dadurch gekennzeichnet, dass** der Brenner (5, 35, 55, 75) und die Hülse (6, 36, 56, 76) mit Hilfe einer porösen Einhüllung zusammen gebaut sind, die so ausgebildet ist, dass die Migration der brennbaren Zusammensetzung (2) der Hülse (6, 36, 56, 76) in Richtung des Brenners (5, 35, 55, 75) gewährleistet ist.

5. System nach einem der Ansprüche 1 bis 3, **dadurch gekennzeichnet, dass** der Brenner (5, 35, 55, 75) und die Hülse (6, 36, 56, 76) ein einziges Stück bilden.

6. System nach einem der Ansprüche 1 bis 5, **dadurch gekennzeichnet, dass** das erste und das zweite poröse Material aus Cordierit hergestellt sind.

7. System nach einem der Ansprüche 1 bis 6, **dadurch gekennzeichnet, dass** der Brenner (35) eine im Wesentlichen axiale Ringkehle (44) aufweist, die sich bis zu der oberen Oberfläche (35c) des Brenners (35) erstreckt und die Umfangszone (39), welche den Katalysator (10) trägt, von der Zentralzone (41) ohne Katalysator, welche die Verdampfungszone bildet, trennt.

8. System nach einem der Ansprüche 1 bis 7, **dadurch gekennzeichnet, dass** der Brenner (5, 35, 55) an seinem unteren Teil (5a, 35a, 55a) einen im Wesentlichen axialen Hohlraum (8, 38, 58) aufweist, der so ausgebildet ist, dass er den Docht (7, 7a) aufnehmen kann.

9. System nach Anspruch 8, **dadurch gekennzeichnet, dass** der Brenner (35, 55) einen Kanal (45, 65) aufweist, um den oberen Teil (38a, 58a) des Hohlraums (38, 58), der den Docht (7, 7a) aufnimmt, mit der Atmosphäre zu verbinden.

10. System nach Anspruch 7 oder 8, wobei das System (3, 30, 50) mit einem Docht (7, 7a) ausgestattet ist, **dadurch gekennzeichnet, dass** der Docht (7, 7a) in den im wesentlichen axialen Hohlraum (12, 42, 62) der Hülse (6, 36, 56) und gegebenenfalls ganz oder teilweise in dem im wesentlichen axialen Hohlraum (8, 38, 58) des Brenners (5, 35, 55) eingespannt ist.

11. Kolben für eine katalytische Verbrennung, der so ausgebildet ist, dass dieser eine brennbare Zusammensetzung (2) aufnehmen kann und an seinem Hals (4) ein Brennersystem für eine katalytische Verbrennung (3, 30, 50, 70) aufnehmen kann, das so ausgebildet ist, dass dieses mit einem Docht (5) zusammen wirkt, der in die brennbare Zusammensetzung (2) eintaucht, **dadurch gekennzeichnet, dass** dieser mit einem Brennersystem (3, 30, 50, 70) gemäß einem der Ansprüche 1 bis 10 ausgestattet ist.

## Claims

1. Catalytic combustion burner system comprising a burner (5, 35, 55, 75) made from a first porous material comprising on its upper part (5b, 35b, 55b, 75b) a peripheral zone (9, 39, 59, 79) supporting a catalyst (10) and a central zone (11, 41, 61, 81) without a catalyst creating a vaporisation zone, **characterised in that** this system (3, 30, 50, 70) furthermore comprises a sleeve (6, 36, 56, 76) made from a second porous material comprising a substantially axial cavity (12, 42, 62, 82) designed to tightly hold a wick (7, 7a) whose purpose is to carry a combustible composition (2) to the burner (5, 35, 55, 75), the said sleeve (6, 36, 56, 76) being placed in line with the lower part (5a, 35a, 55a, 75a) of the burner (5, 35, 55, 75), so that the combustible composition (2) can move from the pores of the upper part (6a, 36a, 56a, 76a) of the sleeve (6, 36, 56, 76) towards the pores of the lower part (5a, 35a, 55a, 75a) of the burner (5, 35, 55, 75).

2. System according to claim 1, **characterised in that** the porosity of the second porous material of the sleeve (6, 36, 56, 76) is preferably less than the porosity of the first porous material of the burner (5, 35, 55, 75).

3. System according to claim 1 or 2, **characterised in that** the sleeve (6, 36, 56, 76) covers at least 10%, generally between 10% and 40%, advantageously between 20% and 30%, and preferably about 25% of the length of the wick (7, 7a).

4. System according to any one of claims 1 to 3, **characterised in that** the burner (5, 35, 55, 75) and the sleeve (6, 36, 56, 76) are assembled via a porous sealing designed to ensure the movement of the combustible composition (2) from the sleeve (6, 36, 56, 76) towards the burner (5, 35, 55, 75).

5. System according to any one of claims 1 to 3, **characterised in that** the burner (5, 35, 55, 75) and the sleeve (6, 36, 56, 76) are one and the same part.

6. System according to any one of claims 1 to 5, **characterised in that** the first and second porous materials are constituted from cordierite.

7. System according to any one of claims 1 to 6, **characterised in that** the burner (35) has a substantially axial ring-shaped groove (44), extending from the upper surface (35c) of the burner (35) and separating the peripheral zone (39) supporting the catalyst (10) from the central zone (41) without a catalyst creating a vaporisation zone.

8. System according to any one of claims 1 to 7, **characterised in that** the burner (5, 35, 55) comprises, on its lower part (5a, 35a, 55a), a substantially axial cavity (8, 38, 58) designed to hold a wick (7, 7a).

9. System according to claim 8, **characterised in that** the burner (35, 55) has a channel (45, 65) to bring the upper part (38a, 58a) of the cavity (38, 58) holding the wick (7, 7a) into contact with the atmosphere.

10. System according to claim 7 or 8, the system (3, 30, 50) being fitted with a wick (7, 7a), **characterised in that** the wick (7, 7a) is inserted into the cavity (12, 42, 62) substantially axial to the sleeve (6, 36, 56) and, if need be, into all or part of the cavity (8, 38, 58) substantially axial to the burner (5, 35, 55).

11. Catalytic combustion flask, designed to contain a combustible composition (2) and to be fitted with, on its neck (4), a catalytic combustion burner system (3, 30, 50, 70) designed to hold a wick (7, 7a) immersed in the said combustible composition (2), **characterised in that** it is fitted with a burner system (3, 30, 50, 70) according to any one of claims 1 to 10.
